# EUROPÄISCHE PATENTANMELDUNG

(11) **EP 1 977 686 A1**
(43) Veröffentlichungstag der Anmeldung: **08.10.2008**
(21) Anmeldenummer: 07105656.8
(22) Anmeldetag: 04.04.2007
(51) Int. Cl.: A61B 5/00, A61B 5/15

(54) **Diagnostischer Einmalartikel**

(71) Anmelder: F.HOFFMANN-LA ROCHE AG, 4070 Basel (CH); Roche Diagnostics GmbH, 68305 Mannheim (DE)
(72) Erfinder: HAAR, Hans-Peter, 69168, Wiesloch (DE)
(74) Vertreter: Pfiz, Thomas

(57) **Zusammenfassung**

Die Erfindung betrifft einen diagnostischen Einmalartikel mit einem in ein Körperteil einstechbaren Stechelement (14) und einem an dem Stechelement (14) ausgebildeten, vorzugsweise kapillaraktiven Sammelbereich (16) für durch den Einstich erhaltene Körperflüssigkeit. Erfindungsgemäß wird vorgeschlagen, dass das Stechelement (14) zwei gegeneinander gefaltete Biegeteile (46) aufweist, und dass die Biegeteile (46) den Sammelbereich (16) zumindest teilweise begrenzen

## Beschreibung

Die Erfindung betrifft einen diagnostischen Einmalartikel insbesondere für Blutzuckertests mit einem in ein Körperteil einstechbaren Stechelement und einem an dem Stechelement ausgebildeten, vorzugsweise kapillaraktiven Sammelbereich für durch den Einstich erhaltene Körperflüssigkeit. Die Erfindung betrifft weiter ein Herstellungsverfahren für eine solche mikrofluidische Sammeleinheit.

Bei in der Regel täglich mehrfach durchgeführten Blutzucker-Selbstkontrollen im Rahmen einer Insulinbehandlung ist es wünschenswert, der betroffenen Person möglichst wenige Handhabungsschritte aufzuerlegen und zugleich eine schmerzarme und zuverlässige Messung sicherzustellen. Dabei werden aus hygienischen Gründen Einmalartikel eingesetzt, die als Massenprodukt möglichst günstig herstellbar sein sollen.

Für eine hohe Systemintegration wurde in der EP-Anmeldung Nr. 0502535.8 bereits ein kombiniertes Testelement vorgeschlagen, bei welchem der Sammelbereich durch eine in Stechrichtung sich erstreckende Ausnehmung gebildet ist, in welcher eine Messzone vorgesehen ist.

Ausgehend hiervon liegt der Erfindung die Aufgabe zugrunde, die im Stand der Technik bekannten Erzeugnisse weiter zu verbessern und so zu gestalten, dass herstellungstechnische Vereinfachungen bei hoher Anwendungsfreundlichkeit erreichbar sind.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den Sammelbereich ohne aufwändige Materialbearbeitungsschritte auszubilden. Dementsprechend wird erfindungsgemäß vorgeschlagen, dass das Stechelement zwei gegeneinander gefaltete Biegeteile aufweist, und dass die Biegeteile den Sammelbereich zumindest teilweise begrenzen. Auf diese Weise wird eine als Sammelvolumen geometrisch abgegrenzte Form erzeugt, ohne dass der Werkstoff abgetragen oder getrennt werden muss. In dem allein durch Biegen gebildeten Volumen kann bevorzugt durch Kapillarkräfte eine möglichst geringe Blutmenge aufgenommen werden, so dass der Benutzer die Probennahme selbst nicht weiter kontrollieren muss.

In vorteilhafter Ausgestaltung ist der Sammelbereich als (bevorzugt in Stechrichtung langgestreckter) Spalt, insbesondere als Kapillarspalt zwischen den Biegeteilen freigehalten.

Um den Flüssigkeitseintritt zu erleichtern, ist es vorteilhaft, wenn der spaltförmige Sammelbereich an seinen quer zur Stechrichtung weisenden Längsrändern einseitig oder beidseitig vorzugsweise durchgängig offen ist. Eine solche Struktur lässt sich durch Falten eines flachen Werkstücks sehr einfach erreichen. Hierbei können die Biegeteile einander gegenüberliegende Begrenzungsflächen für den Sammelbereich aufweisen.

Vorteilhafterweise sind die Biegeteile über eine durch Biegen umgeformte Biegezone des Stechelements miteinander verbunden, so dass ein einheitlicher Körper erhalten bleibt.

Herstellungstechnisch ist es günstig, wenn die Biegezone einen proximalen Endabschnitt des Stechelements bildet. Alternativ ist es auch möglich, dass die Biegezone durch eine in Stechrichtung des Stechelements verlaufende Biegekante gebildet ist, so dass die Biegeteile eine im Querschnitt U-förmige Rinne bilden.

Um eine definierte Stechstruktur zu schaffen, sollten die Biegeteile an ihren ein Stechorgan bildenden Enden über ein Verbindungsmittel vorzugsweise stoffschlüssig miteinander verbunden sein.

Eine weitere Vereinfachung lässt sich dadurch erreichen, dass das Stechelement aus einem vorzugsweise einstückigen Flachmaterialstück, insbesondere einem Blechzuschnitt durch Biegeumformen gebildet ist.

Besonders bevorzugt ist der Sammelbereich in einem proximalen (d.h. von der Stechstelle abgewandten) Abschnitt zugleich als Messzone vorgesehen. In diesem Fall ist als weiteres Bauteil ein zur Erfassung eines Analyten in der gesammelten Körperflüssigkeit ausgebildetes Testelement integriert. Für eine herstellungstechnische Vereinfachung ist es vorteilhaft, wenn das Testelement als vorgefertigter Einsatz in das Stechelement eingesetzt ist. Eine weitere Verbesserung lässt sich dadurch erreichen, dass das Stechelement insbesondere im Bereich der proximalen Biegezone eine Öffnung zum Einführen des Testelements in den Sammelbereich aufweist. Entsprechend sollte der Sammelbereich in einem proximalen Abschnitt eine Aufnahme für das Testelement bilden.

Vorteilhafterweise besitzt das Testelement eine auf den Analyten ansprechende, durch eine Reagenzschicht oder eine Elektrode gebildete Sensorfläche, welche einen Fließquerschnitt des Sammelbereichs begrenzt und somit bereits durch ein mikroskopisches Volumen einer anströmenden Fließfront benetzt werden kann.

Eine weitere Verbesserung der Systemintegration wird dadurch erreicht, dass das Testelement mindestens einen elektrischen oder optischen Signalleiter aufweist, und dass der Signalleiter an einem proximalen Kontaktabschnitt des Testelements mit einer Messelektronik verbindbar ist.

Für eine automatisierte Handhabung ist es vorteilhaft, wenn das Stechelement in einem Trägerteil für eine hin-und hergehende Stechbewegung gehalten ist. Hierbei sollte das Trägerteil eine Aufnahme zum Einführen des Stechelements und ein Kopplungsteil für eine Antriebskopplung aufweist.

Gegenstand der Erfindung ist auch ein diagnostisches System mit einem Gehäuse und einem darin befindlichen Aktuator für einen vorstehend beschriebenen diagnostischen Einmalartikel. Bevorzugt werden derartige Testsysteme in Form eines tragbaren Handgeräts für die Blutzuckerbestimmung oder auch für andere personennahe Überwachungen beispielsweise für die Gerinnungsdiagnostik eingesetzt.

Im Hinblick auf ein Herstellungsverfahren wird die eingangs genannte Aufgabe dadurch gelöst, dass der Sammelbereich durch Biegeumformen des Stechelements zwischen gegeneinander gebogenen Biegeteilen gebildet wird.

Im Folgenden wird die Erfindung anhand der in der Zeichnung schematisch dargestellten Ausführungsbeispiele näher erläutert. Es zeigen
- Fig. 1: ein Blutzuckermessgerät mit einem ein Stechelement umfassenden diagnostischen Einmalartikel in einer schaubildlichen Darstellung;
- Fig. 2: den diagnostischen Einmalartikel nach Fig. 1 in einer perspektivischen Ansicht;
- Fig. 3: eine Explosionsdarstellung des diagnostischen Einmalartikels nach Fig. 2;
- Fig. 4 und 5: ein durch Biegen gebildetes Stechelement des diagnostischen Einmalartikels in zwei Seitenansichten;
- Fig. 6: einen Flachmaterialzuschnitt zur Bildung des Stechelements nach Fig. 4 und 5 in perspektivischer Ansicht;
- Fig. 7: eine weitere Ausführungsform eines Stechelements im Ausgangs- und Endzustand;
- Fig. 8: das mit einem Testelement bestückte Stechelement nach Fig. 7 in perspektivischer Ansicht; und
- Fig. 9: einen diagnostischen Einmalartikel mit einem Stech- und Testelement nach Fig. 8.

Die in der Zeichnung dargestellten diagnostischen Einmalartikel 10 lassen sich als mikrofluidische Sammeleinheiten in einem Handgerät 12 von einem Benutzer vor Ort für eine Blutzuckermessung einsetzen. Zu diesem Zweck umfassen die Einmalartikel 10 ein in einer Stechbewegung in ein Körperteil einstechbares Stechelement 14, einen durch Biegeumformung daran ausgebildeten Sammelbereich 16 zur Aufnahme einer durch den Einstich erhaltenen Blutprobe und ein Testelement 18 zur Erfassung eines Analyten (Glucose) in der Probe. Gegebenenfalls kann die gesammelte Körperflüssigkeit auch auf ein gesondertes Testelement übertragen werden.

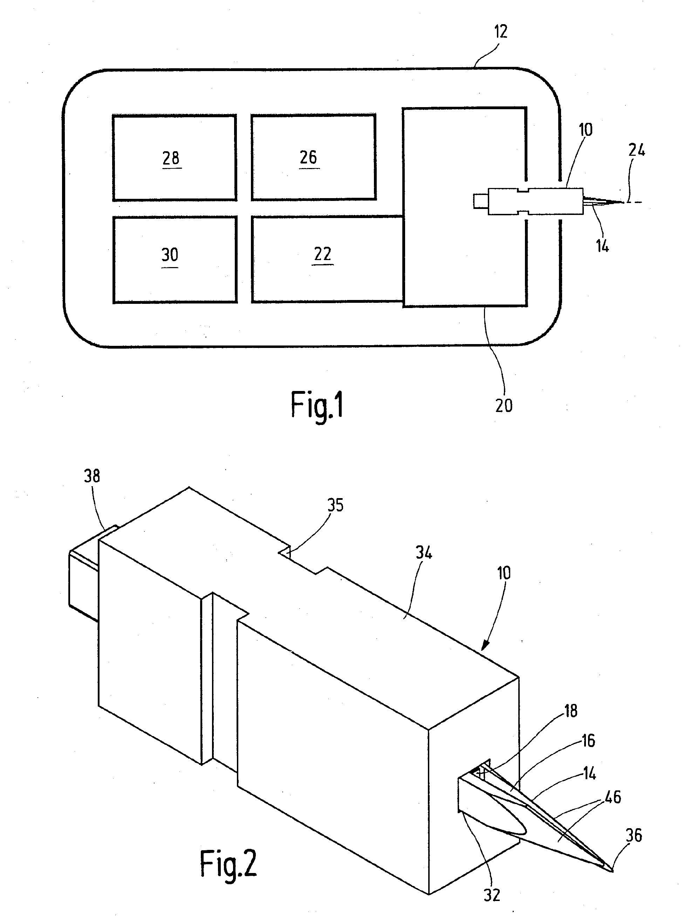

Wie in Fig. 1 veranschaulicht, lassen sich die Sammeleinheiten 10 in einem Magazins 20 sukzessive in eine aktive Gebrauchsposition bringen. Ein in das Magazin 20 eingreifender Aktuator 22 ermöglicht dabei eine hin-und hergehende Stechbewegung der jeweiligen Sammeleinheit 10 in einer Stechachse bzw. Stechrichtung 24. Die über das Testelement 18 gewonnenen Messdaten werden in einer elektronischen Verarbeitungseinheit 26 ausgewertet und über eine Ausgabeeinheit 28 für den Benutzer dargestellt. Eine Energieversorgung 30 ermöglicht einen autarken Betrieb als kompaktes Handgerät, so dass die Selbstbestimmung der Blutzuckerkonzentration in einem vollautomatischen Messablauf auch für Laien mit hohem Handhabungskomfort zuverlässig durchführbar ist. Generell können neben Kapillarblut aus der Haut auch Gewebeflüssigkeit oder Mischungen davon als Probe in Frage kommen

Gemäß Fig. 2 ist das Stechelement 14 in einer Aufnahme 32 eines Trägerteils 34 gehalten, um eine Antriebskopplung beispielsweise über Formschlussmittel 35 mit dem Aktuator 22 zu ermöglichen. Dabei ragt ein Stechorgan 36 des Stechelements 10 in distaler Richtung aus dem Trägerteil 32 heraus, während ein proximal überstehendes Anschlussteil 38 des Testelements 18 eine Signalübertragung ermöglicht. Das Trägerteil 34 kann als Spritzgussformteil aus Kunststoff bestehen.

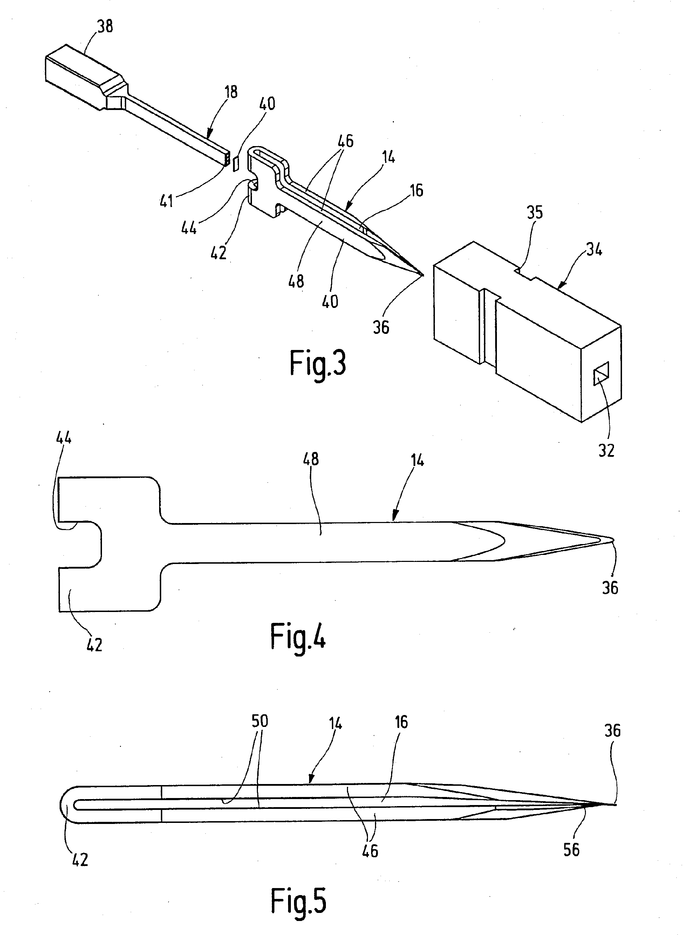

Fig. 3 zeigt die zusammensetzbaren Bauteile der Sammeleinheit 10. Im Einzelnen umfassen diese das Stechelement 14, das Trägerteil 34 und das Testelement 18. Letzteres ist an einer distalen Stirnseite mit einer auf den Analyten ansprechenden Reagenzschicht 40 versehen, die über optische Signalleiter 41 für eine reflektometrische Messung mit dem Anschlussteil 38 verbunden ist. Die Reagenzschicht 40 kann als an sich bekanntes enzymatisches System ausgebildet sein, welches mit Blutglucose unter Farbänderung irreversibel reagiert, sich aber nicht in der Blutflüssigkeit löst. Durch streuende Partikel innerhalb des Chemiesystems wird unter Rückstreuung des über die Lichtleiter 41 eingestrahlten Messlichts eine geräteseitige optische Detektion ermöglicht.

Der stirnseitig beschichtete Schaft des Testelements 18 ist in Stechrichtung in den proximalen Abschnitt des Sammelbereichs 16 einschiebbar, so dass die Reagenzschicht 40 einen Fließquerschnitt frontal begrenzt. Zu diesem Zweck weist das Stechelement 14 im Bereich einer proximalen Biegezone 42 einen in den Sammelbereich 16 mündenden Durchbruch 44 auf.

Wie auch aus Fig. 4 und 5 erkennbar, ist der Sammelbereich 16 durch zwei gegeneinander gebogene bzw. gefaltete Biegeteile 46 des Stechelemements 14 beidseitig begrenzt. Die Biegeteile bzw. Biegepartien 46 bilden dabei einen Stechschaft 48, an dessen distalen Ende eine Spitze als Stechorgan 36 angeschliffen ist. Zwischen den einander gegenüberliegenden Begrenzungsflächen 50 der Biegeteile 46 wird somit ein Kapillarspalt 30 freigehalten, der an seinen quer zur Stechachse 24 weisenden Längsrändern durchgängig offen ist. Dadurch kann eine effektive Flüssigkeitsaufnahme ohne die Gefahr einer Verstopfung durch Zellbestandteile gewährleistet werden. Durch die Vermeidung von Toträumen und größeren Transportstrecken lässt sich das Sammelvolumen auf einige 10 Nanoliter beschränken, wodurch eine schonende und schmerzarme Blutentnahme möglich ist.

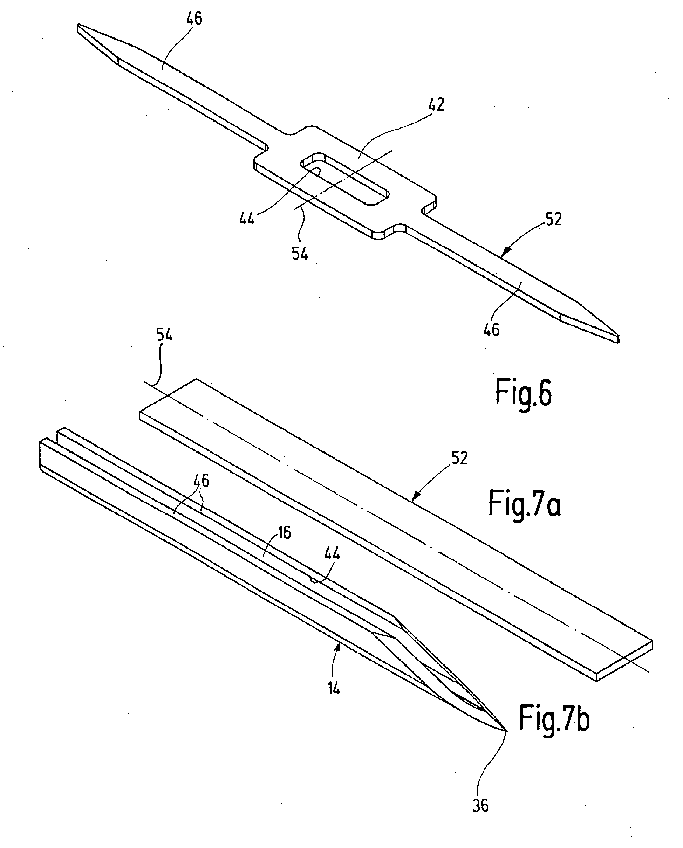

Wie schon erwähnt, ist das Stechelement 14 auf besonders einfache Weise aus einem in Fig. 6 gezeigten Zuschnitt 52 aus Edelstahlblech durch Biegeumformen gebildet. Hierbei werden die beiden symmetrischen Biegeteile 46 um eine quer zur Stechachse 24 mittig verlaufende Biegelinie 54 gebogen, wobei der Biegewinkel jeweils ca. 90° beträgt. Anschließend werden die Spitzen der Biegeteile 46 durch eine Schweißstelle 56 fest miteinander verbunden und so angeschliffen, dass eine scharfe Spitze 36 entsteht.

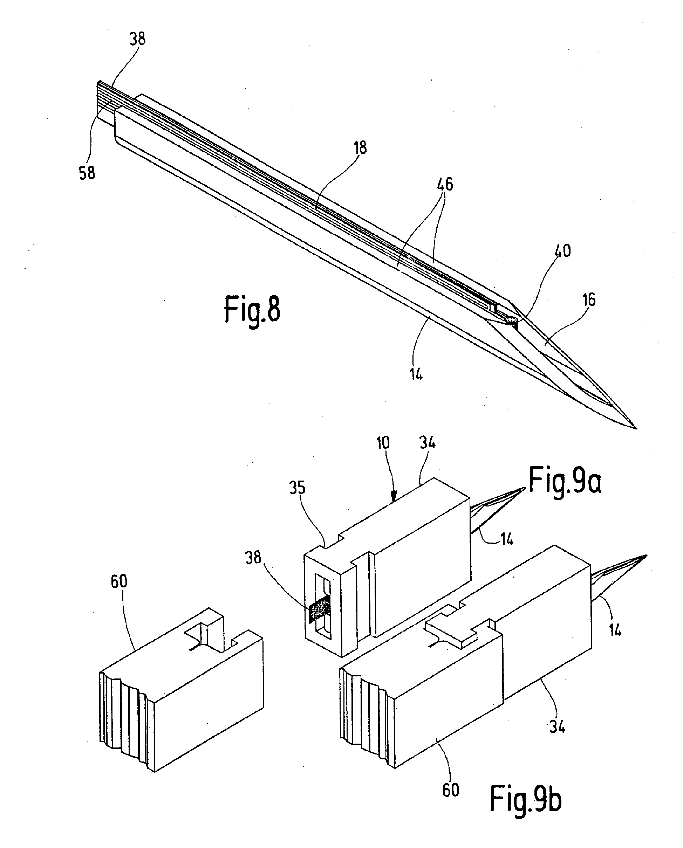

Bei der in Fig. 7 bis 9 gezeigten Ausführungsform sind entsprechende Teile mit gleichen Bezugszeichen versehen. Im Unterschied zu Fig. 6 verläuft hier die Biegelinie 54 in Stechrichtung mittig durch den Flachmaterialzuschnitt 52, so dass die Biegeteile 46 eine im Querschnitt U-förmige Kapillarrinne bilden. In den so begrenzten Sammelbereich 16 lässt sich wiederum ein vorgefertigtes Testelement 18 einsetzen, welches gemäß Fig. 8 durch einen elektrochemisch arbeitenden Teststreifen gebildet sein kann. Hierbei ist ein gegebenenfalls redundant unterteiltes frontales Reagenzfeld 40 über elektrische Leiterbahnen 58 mit dem proximalen Anschlussende 38 verbunden.

Wie in Fig. 9 gezeigt, lässt sich das kombinierte Stech- und Testelement 14, 18 in dem Trägerteil 34 so integrieren, dass die erforderlichen Funktionen einfach realisierbar sind. Über das Formschlussmittel 35 kann die lösbare Antriebskopplung mit einem Greifer 60 des Aktuators 22 hergestellt werden, wobei zugleich ein Signalabgriff am Anschlussende 38 erfolgt. Das Trägerteil 34 ist zweckmäßig ausgestaltet, um auch eine Magazinierung beispielsweise in einem Stapelmagazin 20 zu ermöglichen.

## Patentansprüche

1. Diagnostischer Einmalartikel insbesondere für Blutzuckertests mit einem in ein Körperteil einstechbaren Stechelement (14) und einem an dem Stechelement (14) ausgebildeten, vorzugsweise kapillaraktiven Sammelbereich (16) für durch den Einstich erhaltene Körperflüssigkeit, **dadurch gekennzeichnet, dass** das Stechelement (14) zwei gegeneinander gefaltete Biegeteile (46) aufweist, und dass die Biegeteile (46) den Sammelbereich (16) zumindest teilweise begrenzen.

2. Diagnostischer Einmalartikel nach Anspruch 1, **dadurch gekennzeichnet, dass** der Sammelbereich (16) als Spalt, insbesondere als Kapillarspalt zwischen den Biegeteilen (46) freigehalten ist.

3. Diagnostischer Einmalartikel nach Anspruch 1 oder 2, **dadurch gekennzeichnet, dass** der spaltförmige Sammelbereich (16) an seinen quer zur Stechrichtung weisenden Seitenrändern einseitig oder beidseitig vorzugsweise durchgängig offen ist.

4. Diagnostischer Einmalartikel nach einem der Ansprüche 1 bis 3, **dadurch gekennzeichnet, dass** die Biegeteile (46) einander gegenüberliegende Begrenzungsflächen (50) für den Sammelbereich (16) aufweisen.

5. Diagnostischer Einmalartikel nach einem der Ansprüche 1 bis 4, **dadurch gekennzeichnet, dass** die Biegeteile (46) über eine durch Biegen umgeformte Biegezone (42) des Stechelements (14) miteinander verbunden sind.

6. Diagnostischer Einmalartikel nach Anspruch 5, **dadurch gekennzeichnet, dass** die Biegezone (42) einen proximalen Endabschnitt des Stechelements (14) bildet.

7. Diagnostischer Einmalartikel nach Anspruch 5, **dadurch gekennzeichnet, dass** die Biegezone (42) durch eine in Stechrichtung des Stechelements (14) verlaufende Biegekante gebildet ist, so dass die Biegeteile (46) eine im Querschnitt U-förmige Rinne bilden.

8. Diagnostischer Einmalartikel nach einem der Ansprüche 1 bis 7, durch **gekennzeichnet**, dass die Biegeteile (46) an ihren ein Stechorgan (36) bildenden Enden über ein Verbindungsmittel (56) vorzugsweise stoffschlüssig miteinander verbunden sind.

9. Diagnostischer Einmalartikel nach einem der Ansprüche 1 bis 8, **dadurch gekennzeichnet, dass** das Stechelement (14) aus einem vorzugsweise einstückigen Flachmaterialstück (52), insbesondere einem Blechzuschnitt durch Biegeumformen gebildet ist.

10. Diagnostischer Einmalartikel nach einem der Ansprüche 1 bis 9, **gekennzeichnet durch** ein zur Erfassung eines Analyten in der gesammelten Körperflüssigkeit ausgebildetes Testelement (18).

11. Diagnostischer Einmalartikel nach Anspruch 10, **dadurch gekennzeichnet, dass** das Testelement (18) als vorgefertigter Einsatz in das Stechelement (14) eingesetzt ist.

12. Diagnostischer Einmalartikel nach Anspruch 10 oder 11, durch **gekennzeichnet**, dass das Stechelement (14) insbesondere im Bereich der proximalen Biegezone (42) eine Öffnung (44) zum Einführen des Testelements (18) in den Sammelbereich (16) aufweist.

13. Diagnostischer Einmalartikel nach einem der Ansprüche 10 bis 12, **dadurch gekennzeichnet, dass** der Sammelbereich (16) in einem proximalen Abschnitt eine Aufnahme für das Testelement (18) bildet.

14. Diagnostischer Einmalartikel nach einem der Ansprüche 10 bis 13, **dadurch gekennzeichnet, dass** das Testelement (18) eine auf den Analyten ansprechende, durch eine Reagenzschicht oder eine Elektrode gebildete Sensorfläche (40) aufweist, und dass die Sensorfläche (40) einen Fließquerschnitt des Sammelbereichs (16) begrenzt.

15. Diagnostischer Einmalartikel nach einem der Ansprüche 10 bis 14, **dadurch gekennzeichnet, dass** das Testelement (18) mindestens einen elektrischen oder optischen Signalleiter (41;58) aufweist, und dass der Signalleiter (41;58) an einem proximalen Kontaktabschnitt (38) des Testelements (18) mit einer Messelektronik (26) verbindbar ist.

16. Diagnostischer Einmalartikel nach einem der Ansprüche 1 bis 15, **dadurch gekennzeichnet, dass** das Stechelement (14) in einem Trägerteil (34) für eine hin- und hergehende Stechbewegung gehalten ist.

17. Diagnostischer Einmalartikel nach Anspruch 16, **dadurch gekennzeichnet, dass** das Trägerteil (34) eine Aufnahme (32) zum Einführen des Stechelements (14) und ein Kopplungsteil (35) für eine Antriebskopplung aufweist.

18. Diagnostisches System mit einem Gehäuse (12) und einem darin befindlichen Aktuator (22) für einen diagnostischen Einmalartikel (10) nach einem der vorhergehenden Ansprüche.

19. Verfahren zur Herstellung diagnostischer Einmalartikel (10) insbesondere für Blutzuckertests, bei welchem ein in ein Körperteil einstechbares Stechelement (14) geformt wird, wobei das Stechelement (14) mit einem vorzugsweise kapillaraktiven Sammelbereich (16) für durch den Einstich erhaltene Körperflüssigkeit versehen wird, **dadurch gekennzeichnet, dass** der Sammelbereich (16) durch Biegeumformen des Stechelements (14) zwischen gegeneinander gebogenen Biegeteilen (46) gebildet wird.
